# EUROPÄISCHE PATENTSCHRIFT

(11) **EP 3 587 386 B1**
(45) Veröffentlichungstag und Bekanntmachung des Hinweises auf die Patenterteilung: **19.04.2023**
(21) Anmeldenummer: 18400018.0
(22) Anmeldetag: 22.06.2018
(51) Int. Cl.: C07C 41/09

(54) **VERFAHREN ZUM HERSTELLEN VON FETTALKOHOL-ETHOXYLATEN**
METHOD FOR PRODUCING FAT ALCOHOL ETHOXYLATES
PROCÉDÉ DE PRODUCTION D'ÉTHOXYLATES D'ALCOOLS GRAS

(43) Veröffentlichungstag der Anmeldung: 01.01.2020
(73) Patentinhaber: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Erfinder: Bauer, Ingo, 61118 Bad Vilbel (DE); Cusati, Giuseppe, 60435 Frankfurt am Main (DE); Pötschacher, Peter, 60594 Frankfurt am Main (DE)
(74) Vertreter: Dropsch, Holger

(56) Entgegenhaltungen:
- WO-A1-2013/166985
- WO-A2-2007/113776
- CANDU N ET AL: "AlTf-UVM-7-Highly active catalysts for the synthesis of long chain symmetrical ethers and non-ionic surfactant structures", CHEMICAL ENGINEERING JOURNAL,, Bd. 161, Nr. 3, 15. Juli 2010 (2010-07-15) , Seiten 363-370, XP027103467, ISSN: 1385-8947 [gefunden am 2009-10-20]

## Beschreibung

### Gebiet der Erfindung

Die Erfindung betrifft ein ein Verfahren zur Herstellung von Fettalkohol-Ethoxylaten durch katalytisches Umsetzen des Fettalkohols mit Ethylenglycol, einem Oligo-Ethylenglycol oder einem Poly-Ethylenglycol.

### Stand der Technik

Die Herstellung von Ethoxylaten, die mit der allgemeinen Formel CₙHₘ(OCH₂CH₂)ₓOH beschrieben werden können und eine spezielle Klasse von nichtionischen Tensiden (Niotensiden) darstellen, ist in der Fachwelt an sich bekannt. Die Eigenschaften und Verwendung dieser Tenside wird in der Broschüre "Die fleißigen Verbindungen" des Verbands TEGEWA e. V. 2014, http://www.tegewa.de/uploads/media/Tensid_ Broschuere_2014_deutsch.pdf, abgerufen am 08.05.2018, ausführlich beschrieben.

Wie es in Ullmann's Encyclopedia of Industrial Chemistry, Sixth Edition, 1998 Electronic Release, "Surfactants", Kapitel 7.2 "Ethoxylates", beschrieben wird, werden Ethoxylate im Allgemeinen durch Addition von Ethylenoxid an dissoziierende Protonen enthaltende Verbindungen erhalten.

Als Substrate für die Ethoxylierung werden in erster Linie lineare und verzweigte, primäre und sekundäre C₁₂ bis C₁₈-Alkohole, also beispielsweise natürliche und synthetische Fettalkohole, verwendet. Gemäß Ullmann's Encyclopedia of Industrial Chemistry, Sixth Edition, 1998 Electronic Release, Stichwort "FATTY ALCOHOLS", 1. Introduction, werden aliphatische Alkohole mit Kettenlängen zwischen C₆ und C₂₂ als Fettalkohole bezeichnet. Der Ethoxylierungsgrad, d. h. das Molverhältnis von zugegebenem Ethylenoxid pro Mol Substrat, variiert in weiten Bereichen, im Allgemeinen zwischen 3 und 40, und wird entsprechend der beabsichtigten Verwendung ausgewählt.

Die Addition von Ethylenoxid an ein Substrat, das sauren Wasserstoff enthält, wird durch Basen oder (Lewis)säuren katalysiert. Amphotere Katalysatoren, die in situ hergestellt wurden und wahrscheinlich als feindisperse Feststoffe mit großer Oberfläche existieren, sowie heterogene Katalysatoren wurden ebenfalls beschrieben.

Die Reaktionsmechanismen der basenkatalysierten und säurekatalysierten Ethoxylierung unterscheiden sich, was sich auf die Zusammensetzung der Reaktionsprodukte auswirkt. Bei der basenkatalysierten Ethoxylierung greift ein anfänglich durch Reaktion mit dem Katalysator (Alkalimetall, Alkalimetalloxid, -carbonat, -hydroxid oder -alkoxid) gebildetes Alkoholatanion nucleophil Ethylenoxid an. Das resultierende Anion des Ethylenoxidadditionsprodukts kann eine Gleichgewichtsreaktion mit dem Alkoholausgangsmaterial oder Ethoxylatprodukt eingehen oder kann weiter mit Ethylenoxid reagieren.

Bei der Ethoxylierung von Alkoholen ist die Situation anders. Die Ethersauerstoffatome in Alkyl(oligo)glycolethern erhöhen die Acidität der endständigen primären Hydroxylgruppe im Vergleich zum anfänglichen Alkohol; so gebildete Glycolether reagieren somit bevorzugt mit Ethylenoxid und führen zur Bildung eines Gemischs von homologen Oligoglycolethern, und nicht umgesetzter Ausgangsalkohol verbleibt im Reaktionsgemisch bis zu hohen Ethoxylierungsgraden. Dies gilt insbesondere für die Ethoxylierung von sekundären Alkoholen.

Wenn Lewis-Säuren wie Bortrifluorid, Zinntetrachlorid oder Antimonpentachlorid als Katalysatoren verwendet werden, werden Homologenverteilungen erhalten, die der Poisson-Verteilung angenähert sind.

In dem Artikel von N. Candu et al.: "AITf-UVM-7-Highly active catalysts for the synthesis of long chain symmetrical ethers and non-ionic surfactant structures", CHEMICAL EN-GINEERING JOURNAL, Bd. 161, Nr. 3, 15. Juli 2010 (2010-07-15), Seiten 363-370, werden neue Lewis-Säure-AlTf-UVM-7-Katalysatoren mit bimodalem Porensystem und unterschiedlichen Si/Al-Verhältnissen beschrieben. Diese wurden in einer zweistufigen Synthese hergestellt, bei der Trifluorsäure (Tf) in zuvor synthetisiertes, mesoporöses aluminiumhaltiges Siliciumdioxid eingebaut wurde. Diese Materialien wurden als "grüne" Katalysatoren für die Veretherung von Fettalkoholen und die Umwandlung von Ethylenglycol (EG) mit n-Octanol verwendet, was zu Mischungen mit kurzen ethoxylierten Strukturen mit einer großen Verteilung von C8E1-C8E3-Produkten führte. Der Prozess erfolgte mit hohen Umsätzen von Ethylenglycol und hohen Selektivitäten zu ethoxylierten Produkten. Auch hier wird demnach bei Verwendung von festen Lewis-Säuren als Katalysatoren eine Homologenverteilung für die Ethoxylierungsprodukte erhalten.

Der Umgang mit Ethylenoxid gestaltet sich auf Grund seiner Reaktivität und seiner Giftigkeit problematisch. Zudem bildet Ethylenoxid mit Luft in unbegrenzten Verhältnis zündfähige Dampfgemische, weshalb beim Umgang mit Ethylenoxid ein hoher organisatorischer Aufwand zur Inertisierung von Behältern, Leitungen und Apparaten notwendig wird.

### Beschreibung der Erfindung

Aufgabe der Erfindung ist es daher, ein Verfahren anzugeben, das die genannten Nachteile der aus dem Stand der Technik bekannten Verfahren vermeidet und bei dem insbesondere nur Edukte mit im Vergleich zum Ethylenoxid geringeren Gefahrstoffpotenzial verwendet werden.

Diese Aufgabe wird im Wesentlichen durch ein Verfahren mit den Merkmalen des Anspruchs 1 gelöst. Weitere, insbesondere bevorzugte Ausgestaltungen des erfindungsgemäßen Verfahrens finden sich in den Unteransprüchen.

### Erfindungsgemäßes Verfahren:

Verfahren zur Herstellung von Fettalkohol-Ethoxylaten, dadurch gekennzeichnet, dass der Fettalkohol mit Ethylenglycol, einem Oligo-Ethylenglycol oder einem Poly-Ethylenglycol in Anwesenheit eines aciden Katalysators umgesetzt wird, wobei ein homogener acider Katalysator verwendet wird.

Da Ethylenglycol weniger reaktiv, und somit einfacher zu transportieren und zu behandeln ist, wurde die Möglichkeit geprüft, durch eine Veretherungsreaktion zwischen Ethylenglycol und Fettalkohlen unter Wasserabspaltung zu ethoxylierten Fettalkoholen zu gelangen. Es kann davon ausgegangen werden, dass der Ansatz auch mit anderen Polyolen (kondensierte Ethylenglycole) durchführbar ist.

Durch eine homogen sauer katalysierte Veretherungsreaktion einer Mischung von Ethylenglycol und eines Fettalkohols wurde versucht einen unsymmetrischen Ether (ethoxylierter Fettalkohol) zu synthetisieren, der auf Grund seiner amphiphilen Struktur als oberflächenaktives Produkt dienen könnte. Durch Verwendung eines homogenen Katalysators kann eine gute Vermischung und demzufolge ein guter Kontakt der Reaktanten mit der Katalysatorsubstanz hergestellt werden. Stoffübergangsprobleme werden auf diese Weise vermieden.

### Weitere bevorzugte Ausgestaltungen der Erfindung

Als besonders vorteilhaft hat es sich herausgestellt, wenn als homogener acider Katalysator Methansulfonsäure verwendet wird. Methansulfonsäure ist als Katalysator besonders wirksam und kann über den Handel bezogen werden.

In einem besonderen Aspekt des erfindungsgemäßen Verfahrens liegt die Reaktionstemperatur zwischen 100 und 160 °C, bevorzugt zwischen 130 und 150 °C, wenn als homogener acider Katalysator Methansulfonsäure verwendet wird. Man arbeitet auf diese Weise bei einer hohen Reaktionstemperatur und erreicht folglich hohe Reaktionsgeschwindigkeiten, bleibt aber unterhalb der Zersetzungstemperatur der Methansulfonsäure, die oberhalb von 160 °C liegt.

In einem weiteren besonderen Aspekt des erfindungsgemäßen Verfahrens liegt das Stoffmengenverhältnis von Ethylenglycol zum Fettalkohol zwischen 0,1 bis 10 mol/mol, bevorzugt zwischen 0,5 bis 5 mol/mol, meist bevorzugt zwischen 1 und 3 mol/mol. Auf diese Weise wird ein günstiger Kompromiss zwischen einerseits einer vergleichsweise hohen Selektivität zu dem Fettalkohol-mono-Ethoxylat und andererseits einem hohen Umsatz des Fettalkohols erzielt.

Eine weitere bevorzugte Ausgestaltung des erfindungsgemäßen Verfahrens ist dadurch gekennzeichnet, dass das Reaktionsgemisch nach dem Durchführen der Reaktion abgekühlt und durch Zuführen einer Base neutralisiert wird, wobei sich eine leichte, organische Phase von einer schweren, wässrigen Phase trennt. Nach erfolgter Phasentrennung wird die leichte, organische Phase von der schweren, wässrigen Phase mittels einer Phasentrennvorrichtung abgetrennt, die bevorzugt nach dem Prinzip der Sedimentation, Zentrifugation oder Dekantation arbeitet.

In einem weiteren besonderen Aspekt des erfindungsgemäßen Verfahrens wird die nach Durchführen der Phasentrennung erhaltene, leichte Phase mittels eines thermischen Trennverfahrens aufgearbeitet wird, wobei Fettalkohol-Ethoxylate erhalten werden. Bevorzugt erfolgt dabei die Aufarbeitung mittels Destillation oder Rektifikation.

Es ist günstig, als Edukt ein Oligo-Ethylenglycol mit einer Zahl von 2 bis 15, bevorzugt von 2 bis 8 Ethylenoxideinheiten (-CH₂CH₂O-) zu verwenden, da auf diese Weise technisch und kommerziell interessante Niotenside erzeugt werden können.

### Ausführungs- und Zahlenbeispiel

Weitere Merkmale, Vorteile und Anwendungsmöglichkeiten der Erfindung ergeben sich auch aus der nachfolgenden Beschreibung von Ausführungs- und Zahlenbeispielen. Dabei bilden alle beschriebenen Merkmale für sich oder in beliebiger Kombination den Gegenstand der Erfindung, unabhängig von ihrer Zusammenfassung in den Ansprüchen oder deren Rückbeziehung.

### Ausführungsbeispiel 1

### Reaktion:

Eine Mischung aus 124 g Ethylenglycol (2,0 mol), 130 g n-Octanol (1,0 mol, als Modellsubstanz für einen Fettalkohol) und 25 g 77%ige Methansulfonsäure (entspricht 19,2 g MSA; 0,2 mol) als Katalysator wurde im 1 Liter Rundkolben unter Rühren (Magnetrührer, 1000 U/min⁻¹) auf 90 °C erwärmt. Dabei löste sich die Methansulfonsäure und es stellte sich eine homogene Mischung ein. Die Glasapparatur wurde mit einem Liebig-Kühler ausgestattet, damit sich bildendes Reaktionswasser kondensiert und erfasst werden konnte. Die Kondensationstemperatur betrug ca. 20 °C.

Zu Beginn des Versuches wurde die Heizleistung auf 90 Watt eingestellt und durch ein Gaseinleitungsrohr in die weiterhin agitierte Mischung ein Stickstoffstrom von 500 ml/min eingeleitet. Um deutlich unterhalb der Zersetzungstemperatur der Methansulfonsäure (> 160 °C) zu arbeiten, wurde eine maximale Reaktionstemperatur im Bereich von 140 bis 150 °C angestrebt.

Aufgrund der deutlichen und anhaltenden Kondensatbildung wurde die Reaktion über 7 h betrieben.

Die am Ende der Reaktionszeit erhaltene Kondensatmenge von 26,1 g war 2-phasig und bestand aus 21,5 g unterer wässriger Phase und 4,6 g oberer öliger Phase, welche nicht näher identifiziert wurde. Ein Geruch nach Octanol war nicht ausgeprägt. Die kondensierte Wassermenge hat keinen quantitativen Anspruch, da durch den Stickstoffstrom sowie der Kondensationstemperatur nicht von einer quantitativen Kondensation ausgegangen werden kann.

### Aufarbeitung des Reaktionsprodukts:

Nach Abkühlung des Reaktionsgemisches auf ca. 50°C wurde die gesamte Reaktionsmischung mit 37 g 22%iger Natronlauge (entspricht 29 g Wasser + 8,0 g; 0,2 mol NaOH) gegen Methylorange neutralisiert. Nach der Neutralisation wurde die gesamte Reaktionsmischung von 283 g in einen Scheidetrichter überführt. Es bildeten sich innerhalb von einigen Sekunden zwei Phasen aus, die sich nach der Trennung bei Raumtemperatur (30°C) in 156 g öliger Oberphase und 127 g Unterphase aufteilten.

Von beiden Phasen wurden Mischungen aus jeweils 1 ml Phase und 50 ml Wasser hergestellt und kräftig geschüttelt. Keine von beiden Phasen zeigte eine Schaumbildung.

Die so hergestellte Mischung mit der öligen Oberphase zeigte jedoch ein deutliches Emulgiervermögen, weshalb davon ausgegangen werden konnte, dass sich im Reaktionsgemisch oberflächenaktive Substanzen gebildet und in der öligen Oberphase angereichert hatten. Zur Mischung mit der glycolischen Unterphase und Wasser wurde zusätzlich 1 ml Octanol gegeben und kräftig geschüttelt. Es zeigte sich kein Emulgiervermögen.

Daraufhin wurde die ölige Oberphase weiter aufgearbeitet, indem der nicht umgesetzte Anteil vom Octanol bei 120°C und 15 mbar im Rotationsverdampfer abdestilliert wurde. Als Kondensat wurden 47 g (0,36 mol) Octanol und ca. 2 g Wasser erhalten. Diese Destillation verlief nicht quantitativ, da im Sumpfprodukt immer noch 17 % Octanol analytisch detektiert wurden (siehe Tabelle 1). Dies entsprach einer Menge von 18 g bzw. 0,14 mol Octanol. Somit sind ca. 0,5 mol (0,36 mol + 0,14 mol) des Octanols (entspricht 50 % der Ausgangsmenge von 1 mol) umgesetzt worden.

Nach der Einengung im Rotationsverdampfer wurden 107 g Sumpfprodukt erhalten, welches analysiert wurde. Dazu wurde versucht, die Einzelkomponenten mittels GC-MS zu identifizieren und im Anschluss mittels GC-FID zu quantifizieren. Dies gelang nur teilweise (siehe Tabelle 1). Da ebenfalls keine Standards von Produkten und Nebenprodukten vorlagen, erfolgte die Auswertung über die Area-Prozente, so dass die dargestellten Ergebnisse als halbquantitativ zu bewerten sind.

**Tabelle 1: Analyse der aufgearbeiteten Oberphase**

| **Komponente** | **Retentionszeit (min)** | **Fläche** | **Fläche %** | **Abschätzung Siedebereich (°C)** |
|---|---|---|---|---|
| Ethylenglycol | 15,62 | 143 | 0,9 | 197 |
| 1-Octanol | 19,96 | 2799 | 16,9 | 196 |
| C₁₀H₂₂O₂ | 27,36 | 6688 | 40,4 | 230-240 |
| Unbekannt | 34,40 | 1547 | 9,3 | 270-280 |
| Dioctylether | 36,55 | 3817 | 23,1 | 286-287 |
| Unbekannt | 40,46 | 287 | 1,7 | 310-320 |
| Unbekannt | 42,25 | 1096 | 6,6 | ∼350 |
| Unbekannt | 47,25 | 181 | 1,1 | ∼360 |
| **Summe** | | **16558** | **100** | |

Es wurden ca. 40 % des erwarteten Zielproduktes Ethylenglycol-mono-octylether (C₁₀H₂₂O₂) in der aufgearbeiteten Oberphase identifiziert. Unter Bezugnahme auf die gemessene Masse der Oberphase von 107 g ist es möglich, eine octanolbasierende Selektivitätsaussage zu treffen:
- Es wurden 0,5 mol Octanol umgesetzt (s. o.).
- Davon sind 43,2 g (= 40,4 % * 107 g) bzw. 0,25 mol (Molmasse = 174,3 g/mol) zum Zielprodukt Ethylenglycol-mono-octylether umgesetzt worden, was einer octanolbasierten Selektivität von 50 % entspricht.
- Die Dioctylether-Bildung betrug 24,7 g (= 23,1 %*107 g) bzw. 0,14 mol (Molmasse = 242,5 g/mol) , was einer octanolbasierten Selektivität von 28 % entspricht.
- Es verbleiben ca. 0,11 mol als nicht identifizierbarer, octanolbasierender Verbindungen (Differenz zu 0,5 mol umgesetztem Octanol), was einer octanolbasierten Selektivität von 22 % entspricht.

### Ausführungsbeispiel 2

Molverhältnis Dodecanol, Ethylenglycol, Methansulfonsäure = 1 + 2 + 0,3; Gesamtansatz 500 g.

274,1 g (1,47 mol) Dodecanol wurden zusammen mit 43,3 g Methansulfonsäure (0,44 mol) in den Rundkolben eingewogen und dieser im Abzug in der Heizmanschette platziert. Danach wurde dieser mit einem Liebig-Kühler (Wassertemperatur 20 °C), Thermofühler und einem Gaseinleitungsrohr ausgestattet.

Nach Anstellen des Stickstoffstroms von 0,5 L/min und der Heizung (Zieltemperatur 150 °C) erfolgte das Aufheizen des Reaktionsgemisches unter kräftigen Rühren mittels Magnetrührers. Der Reaktionsbeginn wurde bei Erreichung von 100 °C definiert. Ab diesem Zeitpunkt werden insgesamt 182,6 g Ethylenglycol (2,94 mol) innerhalb der folgenden 6 Stunden kontinuierlich zugegeben (30,4 g/h). Die Zieltemperatur von 150 °C wurde nach 15 min erreicht. Nach vollständiger Zugabe des Ethylenglycols (6 h) wurde die Reaktion noch eine weitere Stunde fortgeführt.

Über die gesamte Reaktionsdauer von 7h wurde eine Kondensatmenge von 95 g gravimetrisch erfasst, welches sich aus 88 g wässriger Unterphase und 7 g organischer Oberphase zusammensetzte. Die Kondensatmenge hat keinen quantitativen Anspruch, da durch den Stickstoffstrom sowie der Kondensationstemperatur nicht von einer quantitativen Kondensation ausgegangen werden kann.

Nach 7 Stunden wurde die Heizung, die Kühlung, der Magnetrührer und die Stickstoffbegasung abgestellt. Das Reaktionsgemisch kühlte somit ab und verblieb über Nacht im Reaktionskolben.

Am nächsten Morgen wurde das Reaktionsgemisch erneut auf 40 °C aufgeheizt, da der Schmelzpunkt des (evtl. nicht umgesetzten) Dodecanols 24 °C beträgt.

Dem verflüssigten Reaktionsgemisch wurde anschließend die zur Methansulfonsäure äquimolare Menge Natriumhydroxid (18,0 g) in Form einer 25 %igen Lösung (72,0 g) langsam unter kräftigen Rühren zugetropft.

Das so weitgehend neutralisierte Reaktionsgemisch wurde zur Phasentrennung in einen Scheidetrichter überführt und bis zur vollständigen Trennung (2 bis 4 h) im Trockenschrank bei 40 °C belassen. Nach erfolgter Trennung wurden 286 g der produkttragenden Oberphase und 150 g der wässrigen/glycolischen Unterphase erhalten.

Die chromatographische Analyse der produkttragenden Oberphase zeigt Tabelle 2.

Die Identifizierung der detektierten Substanzen war nahezu quantitativ (98,5 %). Die Rückrechnung der Wiederfindung der in der produkttragenden Oberphase quantifizierten fettalkoholbasierenden Reaktionsprodukte im Verhältnis zur eingesetzten Menge an Fettalkohol ergab 100 %. Der kalkulierte Wert von 102,4 % ist bedingt durch die Abweichung der semiquantitativen Analytik in area%.

**Tabelle 2: Analyse der aufgearbeiteten Oberphase**

| **Komponente** | | **Kategorie** | **Konz.** | **Wiederfind. FA** |
|---|---|---|---|---|
| | | | **[area %]** | **[mol %]** |
| Ethylenglycol (MEG) | Polyol | Edukt | 0,30 | |
| 1-Dodecanol | Fettalk. (FA) | Edukt | 16,70 | 17,4 |
| Diethylenglycol | MEG-Kond. | Nebenprod. | 0,19 | |
| Triethylenglycol | MEG-Kond. | Nebenprod. | 0,08 | |
| Tetraethylenglycol | MEG-Kond. | Nebenprod. | 0,11 | |
| Dodecyl-mono-ethoxylat | FA-Ethoxylat | Zielprodukt | 15,10 | 12,7 |
| Dodecyl-di-ethoxylat | FA-Ethoxylat | Zielprodukt | 3,10 | 4,4 |
| Dodecyl-tri-ethoxylat | FA-Ethoxylat | Zielprodukt | 1,50 | 2,7 |
| Dodecyl-tetra-ethoxylat | FA-Ethoxylat | Zielprodukt | 0,65 | 1,4 |
| Dodecyl-penta-ethoxylat | FA-Ethoxylat | Zielprodukt | 0,30 | 0,7 |
| Dodecyl-hexa-ethoxylat | FA-Ethoxylat | Zielprodukt | 0,14 | 0,4 |
| Dodecyl-hepta-ethoxylat | FA-Ethoxylat | Zielprodukt | 0,06 | 0,2 |
| Bis-Dodecylether | Bis-Alkylether | Nebenprod. | 52,5 | 57,6 |
| Bis-Dodecyl-ethylenglycol | Bis-Alkylether | Nebenprod. | 5,90 | 2,9 |
| Bis-Dodecyl-diethylenglycol | Bis-Alkylether | Nebenprod. | 0,72 | 0,6 |
| Bis-Dodecyl-triethylenglycol | Bis-Alkylether | Nebenprod. | 0,19 | 0,2 |
| Bis-Dodecyl-tetraethylenglycol | Bis-Alkylether | Nebenprod. | 0,07 | 0,1 |
| Dodecen | Olefine | Nebenprod. | 0,97 | 1,1 |
| | | | | |
| | | **Edukte** | **17,0** | **17,4** |
| | | **Zielprodukte** | **20,8** | **22,5** |
| | | **Nebenprod.** | **60,7** | **62,5** |
| | | *Gesamt:* | *98,5* | *102,4* |

### Ausführungsbeispiel 3

Molverhältnis Dodecanol, Ethylenglycol, Methansulfonsäure = 1 + 8 + 0,9; Gesamtansatz 500 g.

120,8 g (0,65 mol) Dodecanol wurden zusammen mit 57,2 g Methansulfonsäure (0,58 mol) in den Rundkolben eingewogen und dieser im Abzug in der Heizmanschette platziert. Danach wurde dieser mit einem Liebig-Kühler (Wassertemperatur 20 °C), Thermofühler und einem Gaseinleitungsrohr ausgestattet.

Nach Anstellen des Stickstoffstroms von 0,5 L/min und der Heizung (Zieltemperatur 150 °C) erfolgte das Aufheizen des Reaktionsgemisches unter kräftigen Rühren mittels Magnetrührers. Der Reaktionsbeginn wurde bei Erreichung von 100 °C definiert. Ab diesem Zeitpunkt werden insgesamt 322 g Ethylenglycol (5,19 mol) innerhalb der folgenden 6 Stunden kontinuierlich zugegeben (53,7 g/h). Die Zieltemperatur von 150 °C wurde nach 15 min erreicht. Nach vollständiger Zugabe des Ethylenglycols (6 h) wurde die Reaktion noch eine weitere Stunde fortgeführt.

Über die gesamte Reaktionsdauer von 7h wurde eine Kondensatmenge von 259 g gravimetrisch erfasst, welches sich aus 221 g wässriger Unterphase und 38 g organischer Oberphase zusammensetzte. Die Kondensatmenge hat keinen quantitativen Anspruch, da durch den Stickstoffstrom sowie der Kondensationstemperatur nicht von einer quantitativen Kondensation ausgegangen werden kann.

Nach 7 Stunden wurde die Heizung, die Kühlung, der Magnetrührer und die Stickstoffbegasung abgestellt. Das Reaktionsgemisch kühlte somit ab und verblieb über Nacht im Reaktionskolben.

Am nächsten Morgen wurde das Reaktionsgemisch erneut auf 40 °C aufgeheizt, da der Schmelzpunkt des (evtl. nicht umgesetzten) Dodecanols 24 °C beträgt.

Dem verflüssigten Reaktionsgemisch wurde anschließend die zur Methansulfonsäure äquimolare Menge Natriumhydroxid (23,8 g) in Form einer 25%igen Lösung (95,3 g) langsam unter kräftigen Rühren zugetropft.

Das so weitgehend neutralisierte Reaktionsgemisch wurde zur Phasentrennung in einen Scheidetrichter überführt und bis zur vollständigen Trennung (2 bis 4 h) im Trockenschrank bei 40 °C belassen. Nach erfolgter Trennung wurden 110 g der produkttragenden Oberphase und 167 g der wässrigen/glycolischen Unterphase erhalten.

Die chromatographische Analyse der produkttragenden Oberphase zeigt Tabelle 3.

Die Identifizierung der detektierten Substanzen war nahezu quantitativ (96,5 %). Die Rückrechnung der Wiederfindung der in der produkttragenden Oberphase quantifizierten fettalkoholbasierenden Reaktionsprodukte im Verhältnis zur eingesetzten Menge an Fettalkohol ergab ca. 88 %. Dieser im Vergleich zum Ausführungsbeispiel 2 verringerte Wert zeigt, dass unten den hier gewählten Bedingungen mehr fettalkoholbasierende Reaktionsprodukte in der wässrigen/glycolischen Unterphase verblieben sind.

**Tabelle 3: Analyse der aufgearbeiteten Oberphase**

| **Komponente** | | **Kategorie** | **Konz.** | **Wiederfind. FA** |
|---|---|---|---|---|
| | | | **[area %]** | **[mol %]** |
| Ethylenglycol (MEG) | Polyol | Edukt | 0,06 | |
| 1-Dodecanol | Fettalk. (FA) | Edukt | 2,70 | 2,5 |
| Diethylenglycol | MEG-Kond. | Nebenprod. | 0,03 | |
| Triethylenglycol | MEG-Kond. | Nebenprod. | 0,02 | |
| Tetraethylenglycol | MEG-Kond. | Nebenprod. | 0,12 | |
| Dodecyl-mono-ethoxylat | FA-Ethoxylat | Zielprodukt | 9,30 | 6,8 |
| Dodecyl-di-ethoxylat | FA-Ethoxylat | Zielprodukt | 3,10 | 3,8 |
| Dodecyl-tri-ethoxylat | FA-Ethoxylat | Zielprodukt | 1,80 | 2,9 |
| Dodecyl-tetra-ethoxylat | FA-Ethoxylat | Zielprodukt | 0,62 | 1,2 |
| Dodecyl-penta-ethoxylat | FA-Ethoxylat | Zielprodukt | 0,21 | 0,4 |
| Dodecyl-hexa-ethoxylat | FA-Ethoxylat | Zielprodukt | 0,18 | 0,4 |
| Dodecyl-hepta-ethoxylat | FA-Ethoxylat | Zielprodukt | 0,22 | 0,5 |
| Dodecyl-octa-ethoxylat | FA-Ethoxylat | Zielprodukt | 0,16 | 0,4 |
| Dodecyl-nona-ethoxylat | FA-Ethoxylat | Zielprodukt | 0,09 | 0,2 |
| Dodecyl-deca-ethoxylat | FA-Ethoxylat | Zielprodukt | 0,05 | 0,1 |
| Bis-Dodecylether | Bis-Alkylether | Nebenprod. | 63,10 | 60,3 |
| Bis-Dodecyl-ethylenglycol | Bis-Alkylether | Nebenprod. | 10,60 | 4,5 |
| Bis-Dodecyl-diethylenglycol | Bis-Alkylether | Nebenprod. | 1,60 | 1,2 |
| Bis-Dodecyl-triethylenglycol | Bis-Alkylether | Nebenprod. | 0,45 | 0,5 |
| Bis-Dodecyl-tetraethylenglycol | Bis-Alkylether | Nebenprod. | 0,17 | 0,2 |
| Bis-Dodecyl-pentaethylenglyc. | Bis-Alkylether | Nebenprod. | 0,06 | 0,1 |
| Dodecen | Olefine | Nebenprod. | 1,90 | 1,9 |
| | | | | |
| | | **Edukte** | **2,8** | **2,5** |
| | | **Zielprodukte** | **15,7** | **16,9** |
| | | **Nebenprodukte** | **78,0** | **69,0** |
| | | *Gesamt:* | *96,5* | *88,4* |

### Ausführungsbeispiel 4

Molverhältnis Octadecanol, Ethylenglycol, Methansulfonsäure = 1 + 2 + 0,3; Gesamtansatz 500 g

318,9 g (1,18 mol) Octadecanol wurden zusammen mit 34,7 g Methansulfonsäure (0,35 mol) in den Rundkolben eingewogen und dieser im Abzug in der Heizmanschette platziert. Danach wurde dieser mit einem Liebig-Kühler (Wassertemperatur 20 °C), Thermofühler und einem Gaseinleitungsrohr ausgestattet.

Nach Anstellen des Stickstoffstroms von 0,5 L/min und der Heizung (Zieltemperatur 150°C) erfolgte das Aufheizen des Reaktionsgemisches unter kräftigen Rühren mittels Magnetrührers. Der Reaktionsbeginn wurde bei Erreichung von 100 °C definiert. Ab diesem Zeitpunkt werden insgesamt 146,4 g Ethylenglycol (2,36 mol) innerhalb der folgenden 6 Stunden kontinuierlich zugegeben (24,4 g/h). Die Zieltemperatur von 15 0°C wurde nach 15 min erreicht. Nach vollständiger Zugabe des Ethylenglycols (6 h) wurde die Reaktion noch eine weitere Stunde fortgeführt.

Über die gesamte Reaktionsdauer von 7h wurde eine Kondensatmenge von 78,5 g gravimetrisch erfasst, welches sich aus 76,2 g wässriger Unterphase und 2 g organischer Oberphase zusammensetzte. Die Kondensatmenge hat keinen quantitativen Anspruch, da durch den Stickstoffstrom sowie der Kondensationstemperatur nicht von einer quantitativen Kondensation ausgegangen werden kann.

Nach 7 Stunden wurde die Heizung, die Kühlung, der Magnetrührer und die Stickstoffbegasung abgestellt. Das Reaktionsgemisch kühlte somit ab und verblieb über Nacht im Reaktionskolben.

Am nächsten Morgen wurde das Reaktionsgemisch erneut auf 70 °C aufgeheizt, da der Schmelzpunkt des (evtl. nicht umgesetzten) Octadecanols 59 °C beträgt.

Dem verflüssigten Reaktionsgemisch wurde anschließend die zur Methansulfonsäure äquimolare Menge Natriumhydroxid (14,4 g) in Form einer 25 %igen Lösung (57,7 g) langsam unter kräftigen Rühren zugetropft.

Die chromatographische Analyse der produkttragenden Oberphase zeigt Tabelle 4.

**Tabelle 4: Analyse der aufgearbeiteten Oberphase**

| **Komponente** | | **Kategorie** | **Konz.** | **Wiederfind. FA** |
|---|---|---|---|---|
| | | | **[area %]** | **[mol %]** |
| Ethylenglycol (MEG) | Polyol | Edukt | 0,18 | |
| 1-Octadecanol | Fettalkohol (FA) | Edukt | 33,30 | 34,3 |
| Diethylenglycol | MEG-Kondensat | Nebenprodukt | 0,23 | |
| Triethylenglycol | MEG-Kondensat | Nebenprodukt | 0,07 | |
| Tetraethylenglycol | MEG-Kondensat | Nebenprodukt | 0,05 | |
| Octadecyl-mono-ethoxylat | FA-Ethoxylat | Zielprodukt | 9,70 | 8,6 |
| Octadecyl-di-ethoxylat | FA-Ethoxylat | Zielprodukt | 1,40 | 2,2 |
| Octadecyl-tri-ethoxylat | FA-Ethoxylat | Zielprodukt | 0,44 | 0,9 |
| Octadecyl-tetra-ethoxylat | FA-Ethoxylat | Zielprodukt | 0,19 | 0,5 |
| Bis-Octadecylether | Bis-Alkylether | Nebenprodukt | 46,10 | 49,1 |
| Bis-Octadecyl-ethylenglycol | Bis-Alkylether | Nebenprodukt | 3,50 | 1,7 |
| Bis-Octadecyl-diethylenglc. | Bis-Alkylether | Nebenprodukt | 0,35 | 0,3 |
| Octadecen | Olefine | Nebenprodukt | 0,97 | 1,1 |
| | | | | |
| | | **Edukte** | **33,5** | **34,3** |
| | | **Zielprodukte** | **11,7** | **12,2** |
| | | **Nebenprodukte** | **51,3** | **52,2** |
| | | *Gesamt:* | *96,5* | *98,7* |

Das so weitgehend neutralisierte Reaktionsgemisch wurde zur Phasentrennung in einen Scheidetrichter überführt und bis zur vollständigen Trennung (2 bis 4 h) im Trockenschrank bei 70 °C belassen. Nach erfolgter Trennung wurden 328,5 g der produkttragenden Oberphase und 115,5 g der wässrigen/glycolischen Unterphase erhalten.

Die Identifizierung der detektierten Substanzen war nahezu quantitativ (96,5 %). Die Rückrechnung der Wiederfindung der in der produkttragenden Oberphase quantifizierten fettalkoholbasierenden Reaktionsprodukte im Verhältnis zur eingesetzten Menge an Fettalkohol ergab ca. 99 %.

### Gewerbliche Anwendbarkeit

Mit der Erfindung wird ein Verfahren zum Herstellen von Fettalkohol-Ethoxylaten vorgeschlagen, dass sich gegenüber der aus dem Stand der Technik bekannten Ethoxylierung mit Ethylenoxid durch die Verwendung von Edukten mit geringem Gefahrstoffpotenzial auszeichnet. Erfindungsgemäß werden dabei die Fettalkohol-Ethoxylate durch Veretherung mit Ethylenglycol, einem Oligo-Ethylenglycol oder einem Poly-Ethylenglycol in Anwesenheit eines aciden Katalysators erhalten.

## Patentansprüche

1. Verfahren zur Herstellung von Fettalkohol-Ethoxylaten, **dadurch gekennzeichnet, dass** der Fettalkohol mit Ethylenglycol, einem Oligo-Ethylenglycol oder einem Poly-Ethylenglycol in Anwesenheit eines aciden Katalysators umgesetzt wird, wobei ein homogener acider Katalysator verwendet wird.

2. Verfahren nach Anspruch 1, **dadurch gekennzeichnet, dass** als homogener acider Katalysator Methansulfonsäure verwendet wird.

3. Verfahren nach Anspruch 1, **dadurch gekennzeichnet, dass** die Reaktionstemperatur zwischen 100 und 160 °C, bevorzugt zwischen 130 und 150 °C liegt.

4. Verfahren nach einem der vorigen Ansprüche, **dadurch gekennzeichnet, dass** das Stoffmengenverhältnis von Ethylenglycol zum Fettalkohol zwischen 0,1 bis 10 mol/mol, bevorzugt zwischen 0,5 bis 8 mol/mol, meist bevorzugt zwischen 1 und 3 mol/mol liegt.

5. Verfahren nach Anspruch 4, **dadurch gekennzeichnet, dass** das Reaktionsgemisch nach dem Durchführen der Reaktion abgekühlt und durch Zuführen einer Base neutralisiert wird, wobei sich eine leichte, organische Phase von einer schweren, wässrigen Phase trennt.

6. Verfahren nach Anspruch 5, **dadurch gekennzeichnet, dass** die leichte, organische Phase von der schweren, wässrigen Phase mittels einer Phasentrennvorrichtung abgetrennt wird.

7. Verfahren nach Anspruch 6, **dadurch gekennzeichnet, dass** die Phasentrennvorrichtung nach dem Prinzip der Sedimentation, Zentrifugation oder Dekantation arbeitet.

8. Verfahren nach Anspruch 6 oder 7, **dadurch gekennzeichnet, dass** die nach Durchführen der Phasentrennung erhaltene, leichte Phase mittels eines thermischen Trennverfahrens aufgearbeitet wird, wobei Fettalkohol-Ethoxylate erhalten werden.

9. Verfahren nach Anspruch 8, **dadurch gekennzeichnet, dass** die Aufarbeitung mittels Destillation oder Rektifikation erfolgt.

10. Verfahren nach einem der vorigen Ansprüche, **dadurch gekennzeichnet, dass** ein Oligo-Ethylenglycol mit einer Zahl von 2 bis 15, bevorzugt von 2 bis 8 Ethylenoxideinheiten (-CH₂CH₂O-) verwendet wird.

## Claims

1. Process for preparing fatty alcohol ethoxylates, **characterized in that** the fatty alcohol is reacted with ethylene glycol, an oligoethylene glycol or a polyethylene glycol in the presence of an acidic catalyst, wherein a homogeneous acidic catalyst is used.

2. Process according to Claim 1, **characterized in that** the homogeneous acidic catalyst used is methanesulfonic acid.

3. Process according to Claim 1, **characterized in that** the reaction temperature is between 100 and 160°C, preferably between 130 and 150°C.

4. Process according to any of the preceding claims, **characterized in that** the molar ratio of ethylene glycol to the fatty alcohol is between 0.1 and 10 mol/mol, preferably between 0.5 and 8 mol/mol, most preferably between 1 and 3 mol/mol.

5. Process according to Claim 4, **characterized in that** the reaction mixture is cooled after conducting the reaction and is neutralized by adding a base, a light, organic phase separating from a heavy, aqueous phase at the same time.

6. Process according to Claim 5, **characterized in that** the light, organic phase is separated off from the heavy, aqueous phase by means of a phase separation apparatus.

7. Process according to Claim 6, **characterized in that** the phase separation apparatus operates by the principle of sedimentation, centrifugation or decantation.

8. Process according to Claim 6 or 7, **characterized in that** the light; phase obtained after conducting the phase separation is worked up by means of a thermal separation process to obtain fatty alcohol ethoxylates.

9. Process according to Claim 8, **characterized in that** the workup is performed by means of distillation or rectification.

10. Process according to any of the preceding claims, **characterized in that** an oligoethylene glycol having a number of from 2 to 15, preferably from 2 to 8, ethylene oxide units (-CH₂CH₂O-) is used.

## Revendications

1. Procédé de préparation d'éthoxylates d'alcool gras, **caractérisé en ce que** l'alcool gras est mis à réagir avec de l'éthylène, glycol, un oligoéthylèneglycol ou un polyéthylène glycol en présence d'un catalyseur acide, un catalyseur acide homogène étant utilisé.

2. Procédé selon la revendication 1, **caractérisé en ce qu'**en tant que catalyseur acide homogène, de l'acide méthanesulfonique est utilisé.

3. Procédé selon la revendication 1, **caractérisé en ce que** la température de réaction se situe entre 100 et 160 °C, préférablement entre 130 et 150 °C.

4. Procédé selon l'une quelconque des revendications précédentes, **caractérisé en ce que** le rapport de quantités de matière d'éthylène glycol sur alcool gras se situe entre 0,1 et 10 mole/mole, préférablement entre 0,5 et 8 mole/mole, le plus préférablement entre 1 et 3 mole/mole.

5. Procédé selon la revendication 4, **caractérisé en ce que** le mélange réactionnel est refroidi après la réalisation de la réaction et est neutralisé par ajout d'une base, une phase organique, légère se séparant d'une phase aqueuse, lourde.

6. Procédé selon la revendication 5, **caractérisé en ce que** la phase organique, légère est séparée de la phase aqueuse, lourde au moyen d'un dispositif de séparation de phases.

7. Procédé selon la revendication 6, **caractérisé en ce que** le dispositif de séparation de phases travaille selon le principe de la sédimentation, de la centrifugation ou de la décantation.

8. Procédé selon la revendication 6 ou 7, **caractérisé en ce que** la phase légère, obtenue après la réalisation de la séparation de phases est traitée au moyen d'un procédé de séparation thermique, des éthoxylates d'alcool gras étant obtenus.

9. Procédé selon la revendication 8, **caractérisé en ce que** le traitement est réalisé au moyen d'une distillation ou d'une rectification.

10. Procédé selon l'une quelconque des revendications précédentes, **caractérisé en ce qu'**un oligoéthylèneglycol doté d'un nombre de 2 à 15, préférablement de 2 à 8 motifs d'oxyde d'éthylène (-CH₂CH₂O-) est utilisé.
